# EUROPEAN PATENT SPECIFICATION

(11) **EP 2 084 528 B1**
(45) Date of publication and mention of the grant of the patent: **29.08.2012**
(21) Application number: 07874224.4
(22) Date of filing: 22.10.2007
(51) Int. Cl.: G01N 33/53, G01N 33/569

(54) **ASSAYS AND DEVICES FOR IDENTIFYING PATHOGENS**
TESTS UND VORRICHTUNGEN ZUR IDENTIFIZIERUNG VON KRANKHEITSERREGERN
DOSAGES ET DISPOSITIFS POUR IDENTIFIER DES PATHOGÈNES

(30) Priority: 20.10.2006 US 853264 P
(43) Date of publication of application: 05.08.2009
(73) Proprietor: Alere Scarborough, Inc., Scarborough, ME 04074 (US)
(72) Inventor: MOORE, Norman, James, North Berwick, ME 03901 (US)
(74) Representative: Lee, Nicholas John
(86) International application number: PCT/US2007/082096
(87) International publication number: WO 2008/133705

(56) References cited:
- WO-A2-03/064604
- US-A- 5 800 992
- US-A1- 2005 181 464
- US-B2- 6 589 741
- BIYANI M ET AL: "Solid-phase translation and RNA-protein fusion: A novel approach for folding quality control and direct immobilization of proteins using anchored mRNA" NUCLEIC ACIDS RESEARCH 200611 GB LNKD- DOI:10.1093/NAR/GKL771, vol. 34, no. 20, November 2006 (2006-11), XP002576823
- KOBATAKE E ET AL: "Translation of immobilized genetic information by yeast cell-free protein synthesizing system." BIOTECHNOLOGY AND BIOENGINEERING 5 APR 1991 LNKD- PUBMED:18600668, vol. 37, no. 8, 5 April 1991 (1991-04-05), pages 723-728, XP002576824 ISSN: 0006-3592
- BLANCHARD S C ET AL: "tRNA dynamics on the ribosome during translation" PROCEEDINGS OF THE NATIONAL ACADEMY OF SCIENCES OF THE UNITED STATES OF AMERICA 20040831 US LNKD- DOI:10.1073/PNAS.0403884101, vol. 101, no. 35, 31 August 2004 (2004-08-31), pages 12893-12898, XP002576825 ISSN: 0027-8424

## Description

### BACKGROUND OF THE INVENTION

Detection of the bacteria that have infected a subject, including metabolites, nucleic acids, and proteins thereof, is a fundamental component in the diagnosis and treatment of medical disorders, as well as in research. A number of methodologies are currently in use for detection. These methodologies can generally be divided into antibody-based diagnostic assays for proteins, either components of the bacteria or byproducts of the disease, and diagnostic assays for nucleic acids, such as the genetic material encoding a component of the bacteria.

Existing methodologies for nucleic acid detection require a high degree of technical competence for reliability due to the complexity of the reaction conditions (for example, PCR requires thermocycling) and may be extremely sensitive to contamination resulting in false positives; they are difficult to use quantitatively rather than qualitatively and thus their sensitivity is compromised. Further, they often take hours to complete. Methodologies for protein detection generally rely on conjugation of an enzyme, usually to additional components of the assay, to increase signal generation and amplification. The use of these additional ligands increases the noise of the system, with higher background and false positives, and necessitates several levels of control reactions.
WO 03/064604 discloses surface-bound, translationally competent ribosome complexes used to generate a translation profile for mRNA. The ribosomes are generic, not pathogen-specific, to the pool of mRNA being tested.
Biyani et al., (Nucleic Acids Research, 2006m 34:20) describe solid-phase translation and RNA-protein fusion for folding quality control and direct immobilisation of proteins using anchored mRNA.
Kobatake et al., (Biotechnology and Bioengineering, 1991, 37:8, 723-728) describes translation of immobilised genetic information by a yeast cell-free protein synthesising system.
Blanchard et al., (PNAS, 2004, 101:35, 12893-12898) describes tRNA dynamics on the ribosome during translation.

### SUMMARY OF THE INVENTION

The invention is as set out in the claims.

The methods are useful for diagnostic or treatment purposes. Ribosomes are specific to a particular pathogen, and thus the identity or presence of a pathogen in a sample may be determined based on determining whether a polypeptide is able to be produced using a nucleic acid template with ribosomes isolated using a binding agent specific for the pathogen's ribosomes. The methods may be performed at a single temperature, avoiding complex reaction conditions. Further, the amplification of the template by the ribosomes is rapid - at least about 20,000 target molecules are produced every 10 seconds - and is very sensitive. Still further, the amplification reaction itself is generic, with only the nucleic acid template and binding agent used to immobilize the ribosomes differing from test to test based on the pathogen to be detected. Thus, manufacturing of test kits and devices for the practice of the methods could be drastically simplified.

The methods may be incorporated into any test format or device suitable for the practice of the methods.

Further objectives and advantages of the present invention will become apparent as the description proceeds.

### BRIEF DESCRIPTION OF THE DRAWINGS

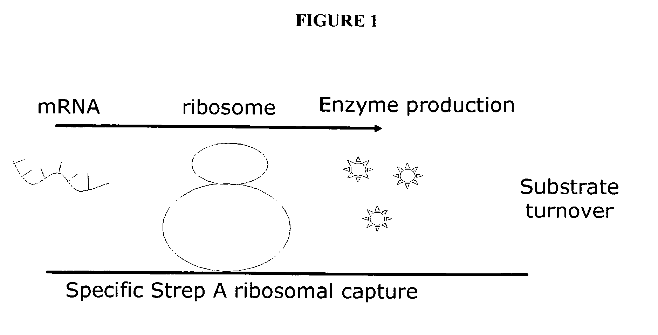

FIGURE 1 depicts a schematic of an exemplary embodiment of the use of ribosomal amplification to detect *Streptococcus* A bacteria.

### DETAILED DESCRIPTION OF THE INVENTION

Unless defined otherwise above, all technical and scientific terms used herein have the same meaning as commonly understood by one of ordinary skill in the art to which this invention belongs. Where a term is provided in the singular, the inventor also contemplates the plural of that term. The nomenclature used herein and the procedures described below are those well known and commonly employed in the art.

The term "amino acid" is intended to embrace all molecules, whether natural or synthetic, which include both an amino functionality and an acid functionality and capable of being included in a polymer of naturally- occurring amino acids. Exemplary amino acids include naturally- occurring amino acids; analogs, derivatives and congeners thereof; amino acid analogs having variant side chains; and all stereoisomers of any of any of the foregoing. The names of the natural amino acids are abbreviated herein in accordance with the recommendations ofIUPAC-IUB.

The term "antibody" refers to an immunoglobulin, derivatives thereof which maintain specific binding ability, and proteins having a binding domain which is homologous or largely homologous to an immunoglobulin binding domain. These proteins may be derived from natural sources, or partly or wholly synthetically produced. An antibody may be monoclonal or polyclonal. The antibody may be a member of any immunoglobulin class, including any of the human classes: IgG, IgM, IgA, IgD, and IgE. In exemplary embodiments, antibodies used with the methods and compositions described herein are derivatives of the IgG class.

The term "antibody fragment" refers to any derivative of an antibody which is less than full-length. In exemplary embodiments, the antibody fragment retains at least a significant portion of the full-length antibody's specific binding ability. Examples of antibody fragments include, but are not limited to, Fab, Fab', F(ab')2, scFv, Fv, dsFv diabody, and Fd fragments. The antibody fragment may be produced by any means. For instance, the antibody fragment may be enzymatically or chemically produced by fragmentation of an intact antibody, it may be recombinantly produced from a gene encoding the partial antibody sequence, or it may be wholly or partially synthetically produced. The antibody fragment may optionally be a single chain antibody fragment. Alternatively, the fragment may comprise multiple chains which are linked together, for instance, by disulfide linkages. The fragment may also optionally be a multimolecular complex. A functional antibody fragment will typically comprise at least about 50 amino acids and more typically will comprise at least about 200 amino acids.

The terms "comprise" and "comprising" is used in the inclusive, open sense, meaning that additional elements may be included.

The term "including" is used herein to mean "including but not limited to". "Including" and "including but not limited to" are used interchangeably.

The term "mRNA" refers to messenger RNA, or the RNA that serves as a template for protein synthesis in a cell. The sequence of a strand of mRNA is based on the sequence of a complementary strand of DNA comprising a sequence coding for the protein to be synthesized.

"Nucleic acid" refers to polynucleotides such as deoxyribonucleic acid (DNA), and, where appropriate, ribonucleic acid (RNA). The term should also be understood to include, as equivalents, analogs of either RNA or DNA made from nucleotide analogs, and, as applicable to the embodiment being described, single (sense or antisense) and double- stranded polynucleotides. ESTs, chromosomes, cDNAs, mRNAs, and rRNAs are representative examples of molecules that may be referred to as nucleic acids.

The term "pathogen" refers to any organism which may cause disease in a subject, such as a bacterium, fungus, parasite, virus, etc.

"Protein" (if single-chain), "polypeptide" and "peptide" are used interchangeably herein when referring to a gene product, e.g., as may be encoded by a coding sequence. When referring to "polypeptide" herein, a person of skill in the art will recognize that a protein can be used instead, unless the context clearly indicates otherwise. A "protein" may also refer to an association of one or more polypeptides.

The term "sample" refers to any sample potentially containing pathogens containing ribosomes.

The term "ribosomal RNA" or "rRNA" refers to the RNA component of ribosome subunits. Ribosomes and their subunits are described further below.

Provided in one aspect is a method, comprising:

(a) capturing ribosomes from a pathogen on a support using a binding agent capable of binding the ribosomes;

(b) incubating the captured ribosomes with a solution comprising mRNA and amino acids; and

(c) detecting the polypeptide produced by the incubated, captured ribosomes and identifying or determining the presence of the pathogen based on the presence of the polypeptide.

Ribosomes are ribonucleoproteins which are present in both prokaryotes and eukaryotes. They comprise about two-thirds RNA and one-third protein. Ribosomes are the cellular organelles responsible for protein synthesis. During gene expression, ribosomes translate the genetic information encoded in a messenger RNA into protein (Garrett et al. (2000) "The Ribosome: Structure, Function, Antibiotics and Cellular Interactions," American Society for Microbiology, Washington, D.C.).

Ribosomes comprise two nonequivalent ribonucleoprotein subunits. The larger subunit (also known as the "large ribosomal subunit") is about twice the size of the smaller subunit (also known as the "small ribosomal subunit"). The small ribosomal subunit binds messenger RNA (mRNA) and mediates the interactions between mRNA and transfer RNA (tRNA) anticodons on which the fidelity of translation depends. The large ribosomal subunit catalyzes peptide bond formation- the peptidyl-transferase reaction of protein synthesis- and includes (at least) two different tRNA binding sites: the A-site which accommodates the incoming aminoacyl-tRNA, which is to contribute its amino acid to the growing peptide chain, and the P-site which accommodates the peptidyl-tRNA complex, i.e., the tRNA linked to all the amino acids that have so far been added to the peptide chain. The large ribosomal subunit also includes one or more binding sites for G-protein factors that assist in the initiation, elongation, and termination phases of protein synthesis. The large and small ribosomal subunits behave independently during the initiation phase of protein synthesis; however, they assemble into complete ribosomes when elongation is about to begin.

Accordingly, as used herein, the term "ribosome" refers to a complex comprising a large ribosomal subunit and small ribosomal subunit. The large ribosomal subunit and small ribosomal subunit are 50 S and 30 S subunits respectively in bacteria and 60 S and 40 S subunits respectively in eukaryotes.

Protocols describing the preparation of samples comprising ribosomes are available in the literature and can be adapted where needed by those skilled in the art. For example, the preparation of ribosomes from bacteria can be done essentially as described by Youmans and Youmans, 1965 and adapted as described by Gregory et al., 1983. In general, but particularly when using virulent Microbes (pathogenic), is recommended to kill the cells prior to further use, for example by treatment with formalin as described by Michalek and McGhee, 1977, and adjust concentrations to 10⁸ bacterial or fungal cells/ml or 10⁷ protozoa/ml. The preparation can be established to be sterile when no multiplication occurs upon inoculation on Sheep blood and Mitis Salivarius agars (DIFCO) or other adapted rich culture medium. Aliquots are stored at -80.degree. C. Subsequently they are thawed rapidly at 37.degrees. C., and 1 g of whole cells is re-suspended with 1 g of micro-glass beads (0.17-0.18 mm) in I ml of PMB to which 3 .mu.g/ml Dnase (SIGMA) is added. The cells are disrupted by shaking for three 2-minute cycles in a Braun homogenizer. Intact cells and debris are removed by two centrifugations (27.000.times.g followed by 47.000.times.g; 10 minutes each).

Preparation of ribosomes from fungi and protozoa follow essentially the same procedure but require adaptation of culture conditions and lysis methods. Given that culture conditions of cultivatable pathogenic microbes are widely available in published literature, preparation of ribosomes from such microbes is well within the possibilities of a person skilled in the art.

Integrity of the ribosomal subunits is important. In particular the stabilization of enclosed large ribosomal RNA's by divalent cations such as provided by MgCl₂, concentration which may need adaptation depending on the microbe and extraction protocol methods used. The ribosomes in the supernatant can be harvested by centrifugation at 180.000 to 250.000.times.g for 2 to 3 hr and then subjected to 5 successive washes in PMB at 180.000 to 250.000.times.g for 2 to 3 hr each. The ribosomal preparation is then clarified twice by two 20-min. centrifugations at 47.000.times.g and the supernatant is filtered through a sterile 0.45 micron Millipore filter (Millipore Filter Corp.). Non-dissociated (=intact) ribosomes can be prepared from gram-negative, Rnase-minus mutant bacteria such as *Escherichia coli* MRE600 following the method of Staehilin et. al., 1969, with modifications as described by M. M. Yusupov and A. S. Spirin. 1988. The preparations can then adjusted to, for example, 20 mg/ml on the basis of protein content by standard protein quantification methods, using, for example, bovine serum albumin as a standard, and maintained at -80.degree. C. until used. Characterization of the ribosomal fraction and purity can be determined by spectral analysis at 235, 280 and 260 nm in order to determine the contamination of ribosomal RNA by DNA polyacrylamide gel electrophoresis permits to evaluate the presence of ribosomal proteins and potential contaminating proteins. The degree of intactness can be evaluated by loading a sample of the original homogenate onto a 10% to 40% sucrose gradient, containing an appropriate concentration of Mg Cl₂ and centrifugation. The elusion profile of the sucrose gradient will show the different fractions: 100S=dimers of 70S ribosomes, 70S=intact ribosomes, 60S=interacting 50S and 30S ribosomal subunits, 50S=large ribosomal subunit, 30S=small ribosomal subunit, material less than 30S=degradation products and contaminants. In good preparations that target non-dissociated ribosomes, the 70S peak contains over 80% of all material. Optionally, the 70S peak containing the target non-dissociated ribosomes may constitute at least 50%, 60%, 70% or 90% of all material.

Pathogens that may be detected using the above methods include any organism comprising ribosomes. Organisms from which ribosomes may be isolated include, but are not limited to, the following:

Ribosomes from bacteria such as: *Acinetobacter calcoaceticus. A. haemolyticus*, *Aeromonas hydrophilia*, *Bacteroides fragilis*, *B. distasonis*, *Bacteroides 3452A homology group*, *B. vulgatus*, *B. ovalus*, *B. thetaiotaomicron*, *B. uniformis*, *B. eggerthii, B. splanchnicus*, *Branhamella catarrhalis. Campylobacterfetus, C. jejuni, C. coli, Citrobacterfreundii, Clostridium difficile, C. diphtheriae, C. ulcerans, C. accolens. C. afermentans, C. amycolatum, C. argentorense, C. auris, C. bovis, C. confusum, C. coyleae, C. durum, C. falsenii, C. glucuronolyticum, C. imitans, C. jeikeium, C. kutscheri. C. kroppenstedtii, C. lipophilum, C. macginleyi, C. matruchoti, C. mucifaciens, C. pilosum, C. propinquum, C. renale, C. riegelii, C. sanguinis. C. singulare, C. striatum, C. sundsvallense, C. thomssenii, C. urealyticum, C. xerosis, Enterobacter cloacae, E. aerogenes. Enterococcus avium, E. casseliflavus, E. cecorum, E. dispar, E. durans, E. faecalis, E. faecium, E. flavescens, E. gallinarum, E. hirae, E. malodoratus, E. mundtii, E. pseudoavium, E. raffinosus, E. solitarius, Francisella tularensis, Gardnerella vaginalis, Helicobacter pylori. Kingella dentrificans, K. kingae, K. oralis, Klebsiella pneumoniae, K. oxytoca, Moraxella catarrhalis, M. atlantae, M. lacunata, M. nonliquefaciens, M. osloensis. M. phenylpyruvica, Morganella morganii, Parachlamydia acanthamoebae, Pasteurella multocida, P. haemolytica, Proteus mirabilis, Proteus vulgaris, Providencia alcalifaciens, P. rettgeri, P. stuartii, Serratia marcescens, Simkania negevensis, Streptococcus pneumoniae, S. agalactiae, S. pyogenes, Treponema pallidum, Vibrio cholerae, and V. parahaemolyticus.*

*Ribosomes from facultative intracellular bacteria such as: Bordetella pertussis, B. parapertussis, B. bronchiseptica, Burkholderia cepacia, Escherichia coli, Haemophilus actinomycetemcomitans, H. aegyptius, H. aphrophilus, H. ducreyi, H. felis, H. haemoglobinophilus, H. haemolyticus, H. influenzae, H. paragallinarum, H. parahaemolyticus, H. parainfluenzae, H. paraphrohaemolyticus, H. paraphrophilus, H. parasuis, H. piscium, H. segnis, H. somnus, H. vaginalis, Legionella adelaidensis, L. anisa, L. beliardensis, L. birminghamensis, L. bozemanii, L. brunensis*, *L. cherrii*, *L. cincinnatiensis*, *Legionella drozanskii L. dumoffli*, *L. erythra*, *L. fairfieldensis*, *L. fallonii*, *L. feeleii*, *L. geestiana*, *L. gormanii*, *L. gratiana*, *L. gresilensis*, *L. hackeliae*, *L. israelensis*, *L. jordanis*, *L. lansingensis*, *Legionella londiniensis L. longbeachae, Legionella lytica L. maceachernii*, *L. micdadei*, *L. moravica*, *L. nautarum*, *L. oakridgensis, L. parisiensis*, *L. pittsburghensis*, *L. pneumophila*, *L. quateirensis*, *L. quinlivanii*, *L. rowbothamii*, *L. rubrilucens*, *L. sainthelensi*, *L. santicrucis, L. shakespearei*, *L. spiritensis*, *L. steigerwaltii*, *L. taurinensis*, *L. tucsonensis*, *L. wadsworthii, L. waltersii*, *L. worsleiensis*, *Listeria denitrificans*, *L. grayi*, *L. innocua*, *L. ivanovii*, *L. monocytogenes*, *L. seeligeri*, *L. welshimeri*, *Mycobacterium abscessus*, *M. africanum*, *M. agri*, *M. aichiense*, *M. alvei*, *M. asiaticum*, *M. aurum*, *M. austroafricanum*, *M. avium, M. bohemicum*, *M. bovis*, *M. branderi, M. brumae, M. celatum, M. chelonae*, *M. chitae*, *M. chlorophenolicum*, *M. chubuense*, *M. confluentis*, *M. conspicuum*, *M. cookii, M. diernhoferi, M. doricum, M. duvalii, M. elephantis*, *M. fallax*, *M. farcinogenes*, *M. flavescens*, *M. fortuitum*, *M. frederiksbergense*, *M. gadium*, *M. gastri*, *M. genavense*, *M. gilvum*, *M. goodii*, *M. gordonae*, *M. haemophilum*, *M. hassiacum*, *M. heckeshornense*, *M. heidelbergense*, *M. hiberniae*, *M. immunogenum*, *M. intracellulare*, *M. interjectum*, *M. intermedium, M. kansasii, M. komossense*, *M. kubicae*, *M. lentiflavum*, *M. leprae*, *M. lepraemurium*, *M. luteum*, *M. madagascariense*, *M. mageritense*, *M. malmoense*, *M. marinum*, *M. microti*, *M. moriokaense*, *M. mucogenicum*, *M. murale*, *M. neoaurum*, *M. nonchromogenicum, M. novocastrense, M. obuense, M. parqfortuitum*, *M. paratuberculosis*, *M. peregrinum*, *M. phage*, *M. phlei*, *M. porcinum*, *M. poriferae*, *M. pulveris*, *M. rhodesiae*, *M. scrofulaceum, M. senegalense*, *M. septicum*, *M. shimoidei*, *M. simiae*, *M. smegmatis*, *M. sphagni*, *M. szulgai*, *M. terrae*, *M. thermoresistibile*, *M. tokaiense*, *M. triplex*, *M. triviale*, *M. tuberculosis*, *M. tusciae*, *M. ulcerans*, *M. vaccae*, *M. wolinskyi*, *M. xenopi*, *Neissenia animalis*, *N. canis*, *N. cinerea*, *N. denitrificans*, *N. dentiae*, *N. elongata*, *N. flava*, *N. flavescens. N. gonorrhoeae, N. iguanae*, *N. lactamica*, *N. macacae*, *N. meninginidis*, *N. mucosa*, *N. ovis*, *N. perflava*, *N. pharyngis var. flava*, *N. polysaccharea*, *N. sicca, N. subflava*, *N. weaveri*, *Pseudomonas aeruginosa*, *P. alcaligenes, P. chlororaphis*, *P. fluorescens*, *P. luteola*, *P. mendocina, P. monteilii*, *P. oryzihabitans*, *P. pertocinogena, P. pseudalcaligenes*, *P. putida, P. stutzeri*, *Salmonella bacteriophage*, *S. bongori, S. choleraesuis. S. enterica*, *S. enteritidis, S. paratyphi*, *S. typhi*, *S. typhimurium*, *S. typhimurium, S. typhimurium, S. typhimurium bacteriophage, Shigella boydii*, *S. dysenteriae*, *S. flexneri*, *S. sonnei, Staphylococcus arlettae*, *S. aureus, S. auricularis*, *S. bacteriophage*, *S. capitis*, *S. caprae*, *S. carnosus, S. caseolyticus*, *S. chromogenes, S. cohnii*, *S. delphini*, *S. epidermidis*, *S. equorum, S. felis, S. fleurettii, S. gallinarum*, *S. haemolyticus, S. hominis, S. hyicus, S. intermedius, S. kloosii, S. lentus, S. lugdunensis*, *S. lutrae, S. muscae, S. mutans, S. pasteuri, S. phage, S. piscifermentans, S. pulvereri, S. saccharolyticus, S. saprophyticus, S. schleiferi, S. sciuri, S. simulans, S. succinus, S. vitulinus, S. warneri, S. xylosus, Ureaplasma urealyticum, Yersinia aldovae, Y. bercovieri, Y. enterocolitica, Y. frederiksenii. Y. intermedia, Y. kristensenii, Y. mollaretii, Y. pestis, Y. philomiragia, Y. pseudotuberculosis, Y. rohdei,* and *Y. ruckeri.*

Ribosomes from obligate intracellular bacteria, such as: *Anaplasma bovis, A. caudatum. A. centrale. A. marginale A. ovis. A. phagocytophila, A. platys, Bartonella bacilliform is, B. clarridgeiae, B. elizabethae, B. henselae, B. henselae phage, B. quintana, B. taylorii, B. vinsonii. Borrelia afzelii, B. andersonii, B. anserina, B. bissettii, B. burgdorferi, B. crocidurae, B. garinii, B. hermsii, B. japonica, B. miyamotoi, B. parkeri, B. recurrentis, B. turdi, B. turicatae, B. valaisiana, Brucella abortus, B. melitensis, Chlamydia pneumoniae. C. psittaci. C. trachomatis, Cowdria ruminantium, Coxiella burnetii, Ehrlichia canis, E. chaffeensis, E. equi, E. ewingii, E. muris, E. phagocytophila, E. platys, E. risticii, E. ruminantium, E. sennetsu, Haemobartonella canis, H. felis, H. muris, Mycoplasma arthriditis, M. buccale, M. faucium, M. fermentans, M. genitalium, M. hominis, M. laidlawii, M. lipophilum. M. orale, M. penetrans, M. pirum, M. pneumoniae. M. salivarium, M. spermatophilum, Rickettsia australis, R. conorii, R. felis, R helvetica, R. japonica, R. massiliae, R. montanensis, R. peacockii, R. prowazekii, R. rhipicephali, R. rickettsii, R. sibirica,* and *R. typhi.*

Ribosomes from facultative intracellular fungi, such as: *Candida Candida aaseri. C. acidothermophilum, C. acutus. C. albicans, C. anatomine, C. apis, C. apis var. galacta, C. atlantica, C. atmospherica, C. auringiensis, C. bertae, C. berthtae var. chiloensis, C. berthetii. C. blankii, C. boidinii, C. boleticola, C. bombi, C. bombicola, C. buinensis. C. butyri, C. cacaoi, C. cantarellii, C. cariosilignicola. C. castellii, C. castrensis, C. catenulata, C. chilensis, C. chiropterorum, C. coipomensis, C. dendronema, C. deserticola, C. diddensiae, C. diversa, C. entomaea, C. entomophila, C. ergatensis, C. ernobii, C. ethanolica, C. ethanothermophilum, C. famata, C. fluviotilis, C. fragariorum, C. fragicola, C. friedrichii. C. fructus, C. geochares, C. glabrata, C. glaebosa, C. gropengiesseri, C. guilliermondii, C. guilliermondii var. galactosa, C. guilliermondii var. soya, C. haemulonii, C. halophila*/*C. versatilis, C. holmii, C. humilis. C. hydrocarbofumarica, C. inconspicua, C. insectalens, C. insectamans, C. intermedia, C. javanica, C. kefyr, C. krissii, C. krusei, C. krusoides, C. lambica, C. lusitaniae, C. mognoliae, C. maltosa, C. mamillae, C. maris, C. maritima, C. melibiosica, C. melinii, C. methylica, C. milleri, C. mogii, C. molischiana, C. montana, C. multis-gemmis, C. musae, C. naeodendra, C. nemodendra, C. nitratophila, C. norvegensis, C. norvegica, C. oleophila, C. oregonensis, C. osornensis, C. paludigena, C. parapsilosis, C. pararugosa, C. periphelosum, C. petrohuensis, C. petrophilum, C. philyla, C. pignaliae, C. pintolopesii var. pintolopesii, C. pintolopesii var. slooffiae, C. pinus, C. polymorpha, C. populi, C. pseudointermedia, C quercitrasa, C. railenensis, C. rhagii, C. rugopelliculosa, C. rugosa, C. sake, C. salmanticensis, C. savonica, C. sequanensis, C. shehatae, C. silvae, C. silvicultrix, C. solani, C. sonorensis, C sorbophila, C*. *spandovensis, C. sphaerica, C. stellata, C. succiphila, C. tenuis, C. terebra, C. tropicalis, C. utilis, C. valida, C. vanderwaltii, C. vartiovaarai, C. veronae, C. vini, C. wickerhamii, C. xestobii, C. zeylanoides,* and *Histoplasma capsulatum.*

Ribosomes from obligate intracellular protozoans, such as: *Brachiola vesicularum, B. connori, Encephalitozoon cuniculi. E. hellem, E. intestinalis, Enterocytozoon bieneusi, Leishmania aethiopica, L amazonensis, L. braziliensis, L chagasi, L. donovani, L. donovani chagasi, L. donovani donovani, L. donovani infantum, L. enriettii, L. guyanensis, L. infantum, L. major, L. mexicana, L. panamensis, L. peruviana, L. pifanoi, L. tarentolae, L. tropica, Microsporidium ceylonensis, M. africanum, Nosema connori, N. ocularum, N. algerae, Plasmodium berghei, P. brasilianum, P. chabaudi, P. chabaudi adami, P. chabaudi chabaudi, P. cynomolgi, P. falciparum, P. fragile, P. gallinaceum, P. knowlesi, P. lophurae, P. malariae, P. ovale, P. reichenowi, P. simiovale, P. simium, P. vinckeipetteri, P. vinckei vinckei, P. vivax, P. yoelii, P. yoelii nigeriensis, P. yoelii yoelii, Pleistophora anguillarum, P. hippoglossoideos, P. mirandellae, P. ovariae, P. typicalis, Septata intestinalis, Toxoplasma gondii, Trachipleistophora hominis, T. anthropophthera, Vittaforma corneae, Trypanosoma avium, T. brucei, T. brucei brucei, T. brucei gambiense, T. brucei rhodesiense, T. cobitis, T. congolense, T. cruzi, T. cyclops, T. equiperdum, T. evansi, T. dionisii, T. godfreyi, T. grayi, T. lewisi, T. mega, T. microti, T. pestanai, T. rangeli, T. rotatorium, T. simiae, T. theileri, T. varani, T. vespertilionis,* and *T. vivax.*

The ribosomes from the pathogen are bound to the support, i.e., immobilized, via a a binding agent. Immobilizing complexes such as ribosomes to a support is well within the skill of one in the art. In certain embodiments, the binding agent is capable of specifically binding a ribosome, e.g. is specific for a ribosome from a particular pathogen. For example, the binding agent may be a polyclonal or monoclonal antibody or an antibody fragment, e.g. specific for the ribosome to be captured. For example, the antibody or antibody fragment may be specific for a ribosomal protein comprising the ribosome. In other embodiments, the binding agent is a ribosomal binding protein specific for the ribosome to be captured. In other embodiments, the binding agent is capable of binding ribosomal RNA, and may be a nucleic acid, i.e., a nucleic acid complementary to and specific for the ribosomal RNA of the ribosome to be captured. In still other embodiments, the binding agent is a protein that binds ribosomal RNA, e.g., an RNA binding protein.

The support may be any suitable material for immobilizing ribosomes via any of the above-described binding agents. In certain embodiments, the support is a porous material, e.g. nitrocellulose. In other embodiments, the support is a particle, e.g., a magnetic particle. In certain embodiments, the support is comprised of a plurality of particles.

In certain embodiments, the support is part of a test strip or test chamber. In some embodiments, the support is a test strip. In other embodiments, the support is part of a lateral flow device. In still other embodiments, the support is resin or other material suitable for packing in a column. In still other embodiments, the support is a plate with wells or a test tube.

The support may be washed with any buffer appropriate to maintaining the biological function of the ribosomes yet sufficient to remove unbound ribosomes and other material. The wash buffer must contain suitable inhibitors of ribosomal activity in order to ensure the residual ribosomes do not translate mRNA. Alternatively, or in addition to adding inhibitors, the wash buffer may be autoclaved to destroy ribosomal activity. Appropriate pH, glycerol or other stabilizing molecules such as ligands, polyethylene glycol, etc., and the presence or absence of reducing agent, chelating agent, cofactors, detergents, protease inhibitors, ribosomal activity inhibitors are accordingly all important considerations. The support may be washed with anywhere from about 5 to 20 volumes of each wash buffer to eliminate unbound ribosomes from the support, e.g., in embodiments wherein the support is or is comprised within a column, lateral flow, or other format wherein the wash may flow over the support. In certain embodiments wherein the support is a particle, the washing may comprise moving the particles from one liquid, e.g., the sample, to another liquid, e.g., the wash.In certain embodiments, the methods described above may be modified to detect viruses. In the case of RNA viruses, a sample potentially comprising an RNA virus may be flowed over a surface comprising antibodies against the virus and ribosomes. As the sample is flowed over the surface, viruses, if present may be captured by the antibodies. After a wash step as described above to remove unbound materials, the viruses may be lysed by flowing a lysis solution over the surface comprising the bound viruses. The lysis releases the RNA, which is available for translation by the ribosomes as follows.

After washing, the support is incubated in the presence of a solution comprising mRNA and amino acids under conditions suitable to allow ribosomes bound to the binding agent to translate the mRNA in the solution into a polypeptide. In certain embodiments, constant temperature is maintained during the incubation.

The solution may include one or more energy sources providing chemical energy for protein synthesis. Further, the solution includes at least one nucleic acid template, for example, an mRNA. The solution may include enzymes, translation factors or co-factors. In certain embodiments, the solution may include *E. coli* rare t-RNAs selected from tRNAs for amino acids arginine, proline, glycine, leucine or isoleucine. Further, the solution may include lipids, cholesterol, or membranes.

The solution may also include an inhibitor of an enzyme that degrades the template or other enzymes necessary for the translation reaction, such as phosphatases, proteases, nucleases, deoxyribonucleases, or ribonucleases. Further, it may include an enzyme to catalyze hydrolysis or formation of phosphodiester bonds.

Still further, the solution may include at least one molecular chaperone or a foldase. The molecular chaperone or foldase includes, but is not limited to, GroEL/ES, GroEL, GroES, TF, DnaK, DnaJ, GrpE, ClpB, FkpA, Skp, DsbA, DsbC, peptidyl prolyl cis/trans isomerase (PPI), chaperonin 60, chaperonin 10, TCP1, TF55, heat shock protein 60, Cpn60, heat shock protein 10, Cpn10, Lim protein, or signal recognition particle.

The amount of protein produced in a translation reaction can be measured by various means, such as specific enzymatic activity, UV or visible light absorption or fluorescence. Products of protein synthesis may also be detected by using antibody based assays. Another method to quantitate the amount of protein produced in a coupled in vitro transcription translation reactions is to perform the reaction using a known quantity of radiolabeled amino acid such as ³⁵S-methionine or ³H-leucine and subsequently measuring the amount of radiolabeled amino acid incorporated into the newly synthesized protein. Incorporation assays will measure the amount of radiolabeled amino acids in all proteins produced in an in vitro translation reaction including truncated protein products.

Other methods for detecting nascent proteins are described in U.S. Published Patent Application 20050032078.

### EXAMPLE

The invention, having been generally described, may be more readily understood by reference to the following examples, which are included merely for purposes of illustration of certain aspects and embodiments of the present invention, and are not intended to limit the invention in any way. All headings are for the convenience of the reader and should not be used to limit the meaning of the text that follows the heading, unless so specified.

FIGURE 1 depicts the use of ribosomal amplification to detect *Streptococcus* A bacteria. Briefly, antibody to a specific ribosomal protein of *Streptococcus* A is bound on a support The sample to be tested is extracted and the bacteria contained within the sample lysed to allow ribosomes to be released (approximately 20,000 ribosomes per bacterial cell). The sample is then flowed over the support to allow the released ribosomes to bind the antibody. The sample is washed to remove unbound ribosomes and other materials from the support, and a generic solution of mRNA encoding an enzyme, amino acids, and substrate is flowed over the support. The ribosomes bound to the antibody should translate mRNA into the enzyme continuously at a consistent temperature compared to PCR. The enzyme upon translation breaks the substrate down, which change may be detected optically.

## Claims

1. A method, comprising:
(a) capturing ribosomes from a pathogen on a support using a binding agent capable of binding the ribosomes;
(b) incubating the captured ribosomes with a solution comprising mRNA and amino acids; and
(c) detecting the polypeptide produced by the incubated, captured ribosomes and identifying or determining the presence of the pathogen based on the presence of the polypeptide.

2. The method of claim 1, wherein the ribosomes are captured by contacting the support with a sample comprising ribosomes and washing the support after contact with the sample, and wherein the support is incubated in the presence of the solution comprising mRNA and amino acids under conditions suitable to allow ribosomes bound to the binding agent to translate the mRNA in the solution into a polypeptide.

3. The method of claim 1, wherein the pathogen is a bacterium.

4. The method of claim 3, wherein the ribosomes are bacterial ribosomes and the method further comprises releasing the ribosomes from at least one bacterium.

5. The method of any preceding claim, wherein the binding agent is capable of specifically binding a ribosome.

6. The method of any preceding claim, wherein the support is a porous material.

7. The method of claim 6, wherein the porous material is nitrocellulose.

8. The method of any preceding claim, wherein the binding agent is an antibody.

9. The method of any preceding claim, wherein the binding agent is an agent capable of binding ribosomal RNA.

10. The method of claim 9, wherein the agent capable of binding ribosomal RNA is a nucleic acid.

11. The method of any preceding claim, wherein the solution comprising mRNA and amino acids further comprises a substrate.

12. The method of claim 11, wherein the polypeptide is an enzyme.

13. The method of claim 12, wherein detecting the polypeptide comprises determining the presence of a reaction product resulting from reaction of the enzyme with a substrate for the enzyme.

14. The method of claim 13, wherein detecting the polypeptide comprises optically detecting the presence of the reaction product.

## Patentansprüche

1. Verfahren, enthaltend:
(a) Erfassen von Ribosomen von einem Pathogen auf einem Träger unter Verwendung eines Bindungsmittels, das zum Binden der Ribosomen in der Lage ist;
(b) Inkubieren der erfassten Ribosomen mit einer Lösung, welche mRNA und Aminosäuren enthält, und
(c) Detektieren des von den inkubierten, erfassten Ribosomen produzierten Polypeptids und Identifizieren oder Bestimmen des Vorhandenseins des Pathogens basierend auf dem Vorhandensein des Polypeptids.

2. Verfahren nach Anspruch 1, wobei die Ribosomen durch In-Kontakt-Bringen des Trägers mit einer Ribosomen enthaltenden Probe und Waschen des Trägers nach Kontakt mit der Probe erfasst werden und wobei der Träger in Gegenwart der mRNA und Aminosäuren enthaltenden Lösung unter Bedingungen inkubiert wird, welche geeignet sind, damit Ribosomen, die an dem Bindungsmittel gebunden sind, die mRNA in der Lösung zu einem Polypeptid translatieren können.

3. Verfahren nach Anspruch 1, wobei es sich bei dem Pathogen um ein Bakterium handelt.

4. Verfahren nach Anspruch 3, wobei die Ribosomen bakterielle Ribosomen sind und das Verfahren des Weiteren ein Freisetzen der Ribosomen aus mindestens einem Bakterium enthält.

5. Verfahren nach einem der vorherigen Ansprüche, wobei das Bindungsmittel zum spezifischen Binden eines Ribosoms in der Lage ist.

6. Verfahren nach einem der vorherigen Ansprüche, wobei es sich bei dem Träger um ein poröses Material handelt.

7. Verfahren nach Anspruch 6, wobei es sich bei dem porösen Material um Nitrocellulose handelt.

8. Verfahren nach einem der vorherigen Ansprüche, wobei es sich bei dem Bindungsmittel um einen Antikörper handelt.

9. Verfahren nach einem der vorherigen Ansprüche, wobei es sich bei dem Bindungsmittel um ein Mittel handelt, das zum Binden von ribosomaler RNA in der Lage ist.

10. Verfahren nach Anspruch 9, wobei es sich bei dem Mittel, das zum Binden von ribosomaler RNA in der Lage ist, um eine Nukleinsäure handelt.

11. Verfahren nach einem der vorherigen Ansprüche, wobei die Lösung, welche mRNA und Aminosäuren enthält, des Weiteren ein Substrat enthält.

12. Verfahren nach Anspruch 11, wobei das Polypeptid ein Enzym ist.

13. Verfahren nach Anspruch 12, wobei das Detektieren des Polypeptids das Feststellen des Vorhandenseins eines Reaktionsproduktes, das aus einer Reaktion des Enzyms mit einem Substrat des Enzyms hervorgeht, enthält.

14. Verfahren nach Anspruch 13, wobei das Detektieren des Polypeptids ein optisches Detektieren des Vorhandenseins des Reaktionsproduktes enthält.

## Revendications

1. Procédé, comprenant :
(a) la capture des ribosomes d'un pathogène sur un support en utilisant un agent de liaison capable de se lier aux ribosomes ;
(b) l'incubation des ribosomes capturés dans une solution comprenant de l'ARNm et des acides aminés ; et
(c) la détection du polypeptide produit par les ribosomes capturés incubés et l'identification ou la détermination de la présence du pathogène en se basant sur la présence du polypeptide.

2. Procédé selon la revendication 1, dans lequel les ribosomes sont capturés en mettant en contact le support avec un échantillon comprenant les ribosomes et en lavant le support après la mise en contact de celui-ci avec l'échantillon, et dans lequel le support est incubé en présence de la solution comprenant l'ARNm et les acides aminés dans des conditions appropriées pour permettre aux ribosomes de se lier à l'agent de liaison afin de traduire l'ARNm présent dans la solution en un polypeptide.

3. Procédé selon la revendication 1, dans lequel le pathogène est une bactérie.

4. Procédé selon la revendication 3, dans lequel les ribosomes sont des ribosomes de bactérie et le procédé comprenant en outre la libération des ribosomes d'au moins une bactérie.

5. Procédé selon l'une quelconque des revendications précédentes, dans lequel l'agent de liaison est capable de se lier spécifiquement à un ribosome.

6. Procédé selon l'une quelconque des revendications précédentes, dans lequel le support est un matériau poreux.

7. Procédé selon la revendication 6, dans lequel le matériau poreux est de la nitrocellulose.

8. Procédé selon l'une quelconque des revendications précédentes, dans lequel l'agent de liaison est un anticorps.

9. Procédé selon l'une quelconque des revendications précédentes, dans lequel l'agent de liaison est un agent capable de se lier à un ARN ribosomal.

10. Procédé selon la revendication 9, dans lequel l'agent capable de se lier à l'ARN ribosomal est un acide nucléique.

11. Procédé selon l'une quelconque des revendications précédentes, dans lequel la solution comprenant l'ARN et les acides aminés comprend en outre un substrat.

12. Procédé selon la revendication 11, dans lequel le polypeptide est une enzyme.

13. Procédé selon la revendication 12, dans lequel la détection du polypeptide comprend la détermination de la présence d'un produit de réaction résultant de la réaction de l'enzyme avec un substrat de l'enzyme.

14. Procédé selon la revendication 13, dans lequel la détection du polypeptide comprend la détection optique de la présence du produit de la réaction.
